# EUROPEAN PATENT APPLICATION

(11) **EP 4 806 988 A1**
(43) Date of publication of application: **16.09.2026**
(21) Application number: 24889160.8
(22) Date of filing: 07.11.2024
(51) Int. Cl.: C12N 5/0775, A61K 35/28, A61P 11/00

(54) **COMPOSITION FOR PREVENTING OR TREATING ACUTE RESPIRATORY DISTRESS SYNDROME COMPRISING PRIMED MESENCHYMAL STEM CELLS AS ACTIVE INGREDIENT**

(30) Priority: 07.11.2023 KR 20230152844
(71) Applicant: Kangstem Biotech Co., Ltd., Seoul 06179 (KR)
(72) Inventor: KONG, Taeho, Seongnam-si Gyeonggi-do 13256 (KR); SEO, Sukyoung, Seoul 07964 (KR); HAN, Yongseok, Paju-si Gyeonggi-do 10823 (KR); KYUNG, Jae Won, Seoul 03943 (KR); SEO, Woo Min, Seoul 08523 (KR); LEE, Seunghee, Seoul 08797 (KR)
(74) Representative: Regimbeau
(86) International application number: PCT/KR2024/017560
(87) International publication number: WO 2025/100974

(57) **Abstract**

The present invention relates to a composition for preventing or treating acute respiratory distress syndrome, comprising a priming mesenchymal stem cell as an active ingredient.

## Description

### [Technical Field]

The present invention relates to a composition for preventing or treating acute respiratory distress syndrome, comprising a primed mesenchymal stem cell as an active ingredient.

### [Background Art]

Acute respiratory distress syndrome (ARDS) and acute lung injury (ALI) often result from infection, trauma, or other systemic inflammatory responses. The major characteristics of ARDS correspond to life-threatening respiratory failure due to widespread lung inflammation, with an acute onset of inflammation and increased permeability of the alveolar-capillary barrier leading to respiratory dysfunction.

Recent research and clinical trials suggest novel treatments for ARDS. For example, corticosteroids suppress inflammatory responses. In particular, steroids such as dexamethasone have been shown to reduce inflammation and increase patient survival. Tocilizumab, an interleukin-6 (IL-6) inhibitor used for COVID-19-related ARDS, also controls inflammation and may thus have therapeutic benefits. However, current treatments are mainly adjunctive therapies and do not fully address the underlying pathology.

With these limitations, another emerging treatment is mesenchymal stem cell (MSC)-based therapy, which has shown promising results in treating ARDS. One of the major properties of MSCs is their potent immunomodulatory and anti-inflammatory effects, which hold significant promise for the treatment of severe inflammatory diseases such as ARDS.

MSCs interact with and regulate the activity of a variety of immune cells, including T cells, B cells, natural killer (NK) cells, and dendritic cells. For example, MSCs can suppress the activity of T cells and promote the production of regulatory T cells (Treg) to mitigate an overactive immune response. Additionally, MSCs modulate the inflammatory response by reducing the secretion of pro-inflammatory cytokines (TNF-α, IL-1β, and IL-6) and secreting anti-inflammatory cytokines (TGF-α and IL-10).

Recently, methods have been studied for achieving higher efficacy in targeted therapy by manipulating MSCs to enhance specific functions through a priming method, which increases therapeutic effects by applying specific stimuli, such as cytokines, hypoxic conditions, or chemical stimulation, to the MSCs, thereby providing MSCs with therapeutic properties superior to those of unprimed MSCs. However, the mechanisms by which MSCs exert therapeutic effects in ARDS remain unclear, and therefore, there is a significant lack of detailed research on the application of priming MSCs to ARDS.

### [Disclosure]

### [Technical Problem]

The present inventors have newly identified that a composition for enhancing immunomodulatory ability of a priming mesenchymal stem cell, comprising IL-1β, IFN-γ, or a combination thereof as an active ingredient, and a pharmaceutical composition comprising the same can replace existing immunosuppressive and inflammatory agents known to have adverse side effects and are significantly superior in preventing or treating ARDS, thereby completing the present invention.

### [Technical Solution]

One object of the present invention is to provide a composition for enhancing immunomodulatory ability of a priming mesenchymal stem cell, comprising IL-1β, IFN-γ, or a combination thereof as an active ingredient.

Another object of the present invention is to provide a pharmaceutical composition for preventing or treating acute respiratory distress syndrome, comprising the composition for enhancing immunomodulatory ability.

Still another object of the present invention is to provide a method for preventing or treating acute respiratory distress syndrome, comprising administering the pharmaceutical composition to an individual other than a human.

Still another object of the present invention is to provide a composition for preparing a priming mesenchymal stem cell having enhanced immunomodulatory ability, comprising IL-1β, IFN-γ, or a combination thereof as an active ingredient.

Still another object of the present invention is to provide a method for preparing a priming mesenchymal stem cell having enhanced immunomodulatory ability, comprising treating a mesenchymal stem cell with IL-1β, IFN-γ, or a combination thereof.

Still another object of the present invention is to provide a priming mesenchymal stem cell having enhanced immunomodulatory ability prepared by the method.

Still another object of the present invention is to provide a pharmaceutical composition for preventing or treating acute respiratory distress syndrome, comprising, as an active ingredient, a priming mesenchymal stem cell having enhanced immunomodulatory ability prepared by the method.

Still another object of the present invention is to provide a use of a composition for enhancing immunomodulatory ability of a priming mesenchymal stem cell, the composition comprising IL-1β, IFN-γ, or a combination thereof as an active ingredient.

Still another object of the present invention is to provide a method for enhancing immunomodulatory ability of a priming mesenchymal stem cell, comprising treating a mesenchymal stem cell with a composition comprising IL-1β, IFN-γ, or a combination thereof as an active ingredient.

Still another object of the present invention is to provide a use of a pharmaceutical composition for preventing or treating acute respiratory distress syndrome, the pharmaceutical composition comprising the composition for enhancing immunomodulatory ability.

Still another object of the present invention is to provide a use of a composition for preparing a priming mesenchymal stem cell having enhanced immunomodulatory ability, the composition comprising IL-1β, IFN-γ, or a combination thereof as an active ingredient.

### [Advantageous Effects]

The priming mesenchymal stem cell having enhanced immunomodulatory ability according to the present invention or a pharmaceutical composition comprising the same are economically available cell therapeutics that are free from side effects. They are expected to replace existing immunosuppressants and inflammatory agents known to have side effects, and to be effectively used for the prevention or treatment of ARDS.

### [Brief Description of the Drawings]

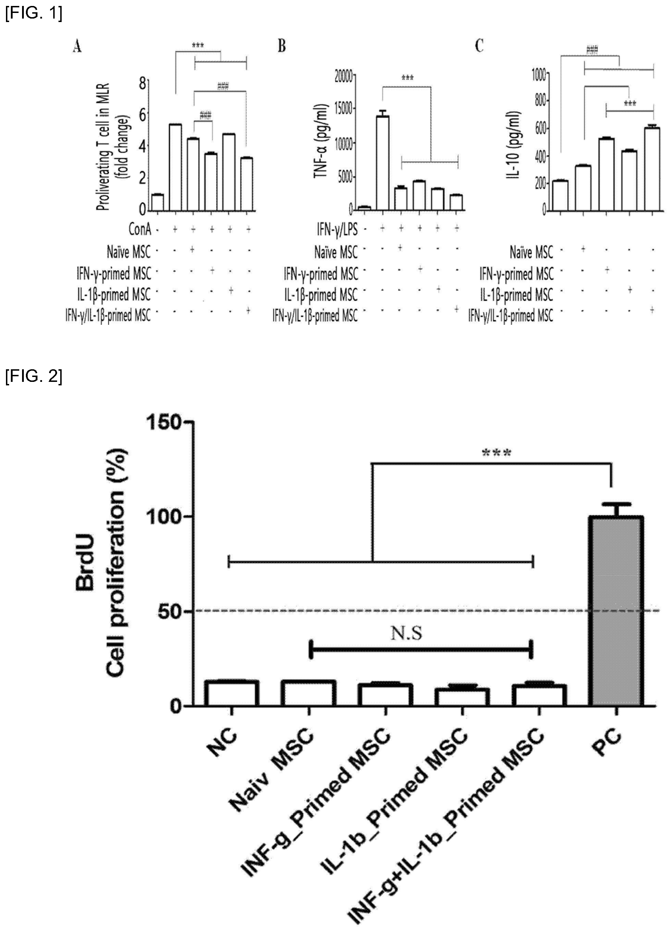

FIG. 1 is a figure showing that co-treated priming MSC prepared according to one embodiment of the present invention exhibits improved immunomodulatory ability compared to pure MSC. Specifically, (A) is the result of confirming the immunomodulatory ability of pure MSCs and priming MSCs using direct and indirect co-culture methods with IFN-γ or IL-1β, (B) is the result of confirming TNF-α secretion ability in M1 macrophage, and (C) is the result of confirming IL-10 secretion ability in M2 macrophage.
FIG. 2 is a figure showing the immunogenicity evaluation of pure MSC and co-treated priming MSC prepared according to one embodiment of the present invention through MLR analysis.

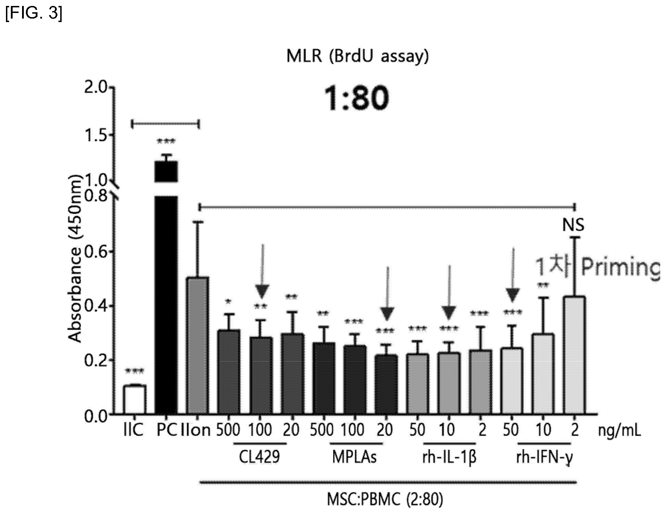

FIG. 3 is a figure confirming the treatment concentrations and times at which immunomodulatory ability is exhibited without showing immunogenicity of IFN-γ and IL-1β.

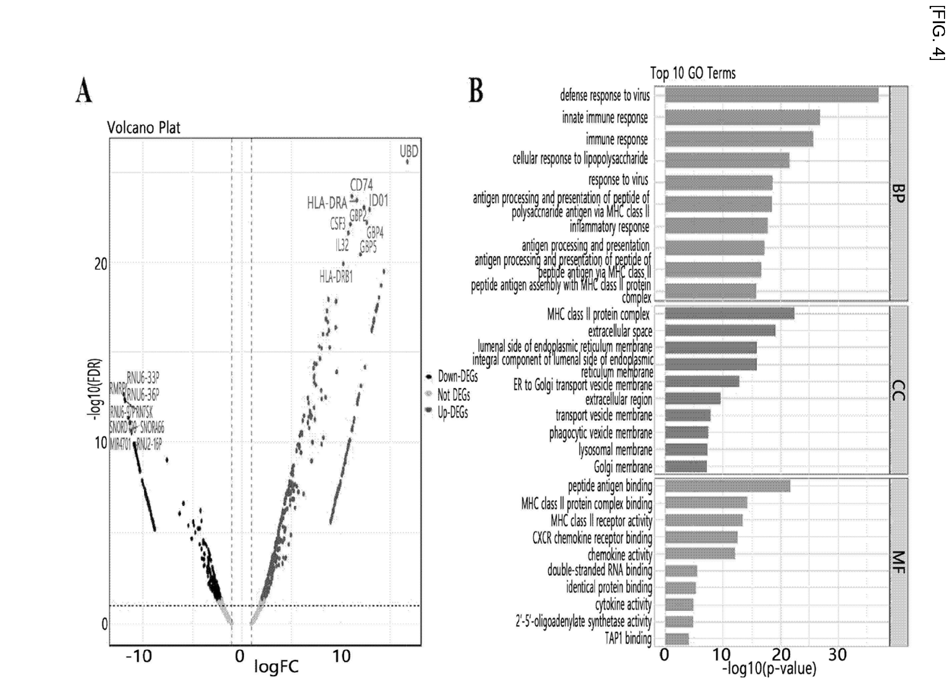

FIG. 4 is a figure showing the results of RNA analysis of co-treated priming MSC prepared according to one embodiment of the present invention and pure MSC. Specifically, (A) is the result showing the gene expression distribution between two conditions using a volcano plot, and (B) is the result of deriving the top 10 terms for classification and distribution analysis of upregulated DEGs through GO analysis.

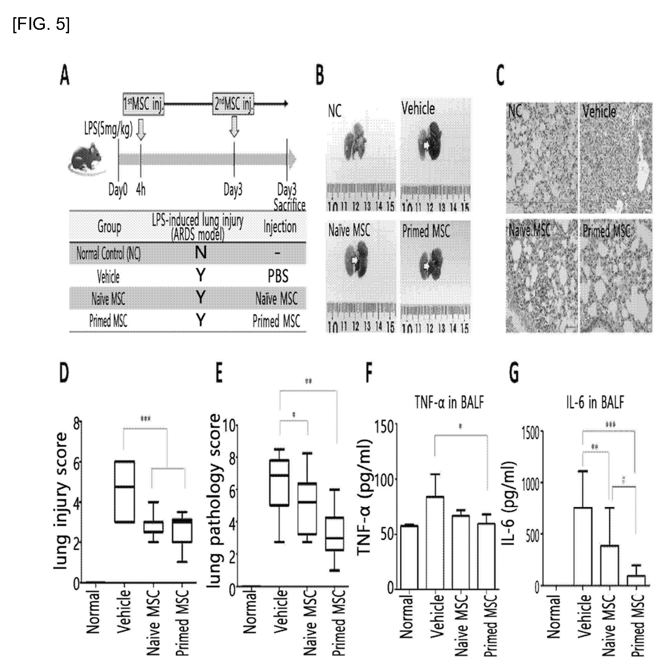

FIG. 5 is a figure showing the effect of co-treated priming MSC prepared according to one embodiment of the present invention on alleviating pulmonary pathological effects in ARDS induced by LPS administration. Specifically, (A) is the experimental design, (B) is a representative image of whole lungs of all experimental groups, (C) is the H&E staining result for lung sections of each group, (D) is the quantitative analysis result of lung injury score, (E) is the lung pathology score analysis, (E) is the measurement of TNF-α concentration in the BALF of each group, and (F) is the measurement result of IL-6 concentration in the BALF of each group.

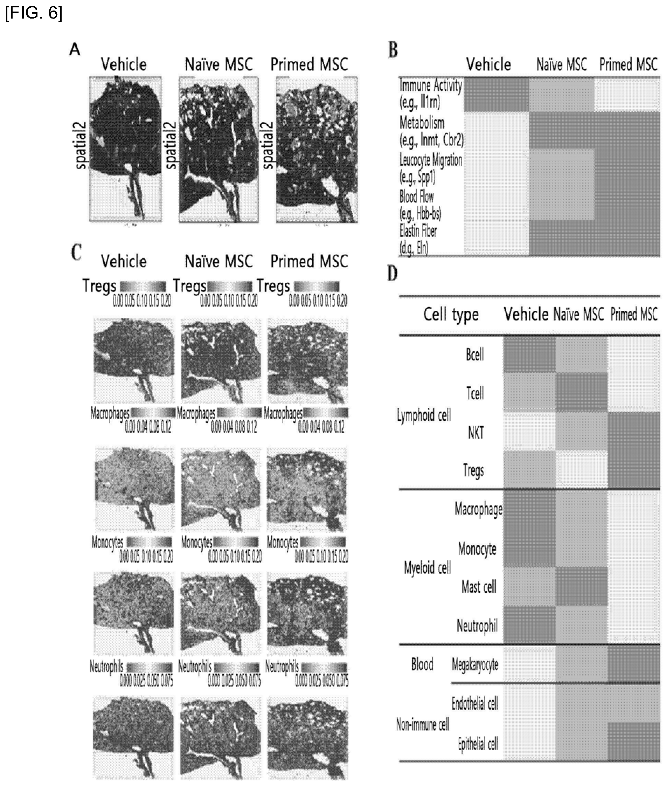

FIG. 6 is a figure showing the distribution and immunosuppressive effect of pure MSC and co-treated primed MSC prepared according to one embodiment of the present invention. Specifically, (A) is the result of evaluating the distribution of pure MSCs and priming MSCs in the lungs by analyzing human gene transcripts (yellow), (B) is the result of confirming the immunosuppressive effects of pure MSCs and priming MSCs through human cluster analysis, (C) is the result of analyzing immune cell and stromal cell types through cell type abundance analysis (xCell), and (D) is the result of comparing mouse cell type abundance between groups through xCell analysis.

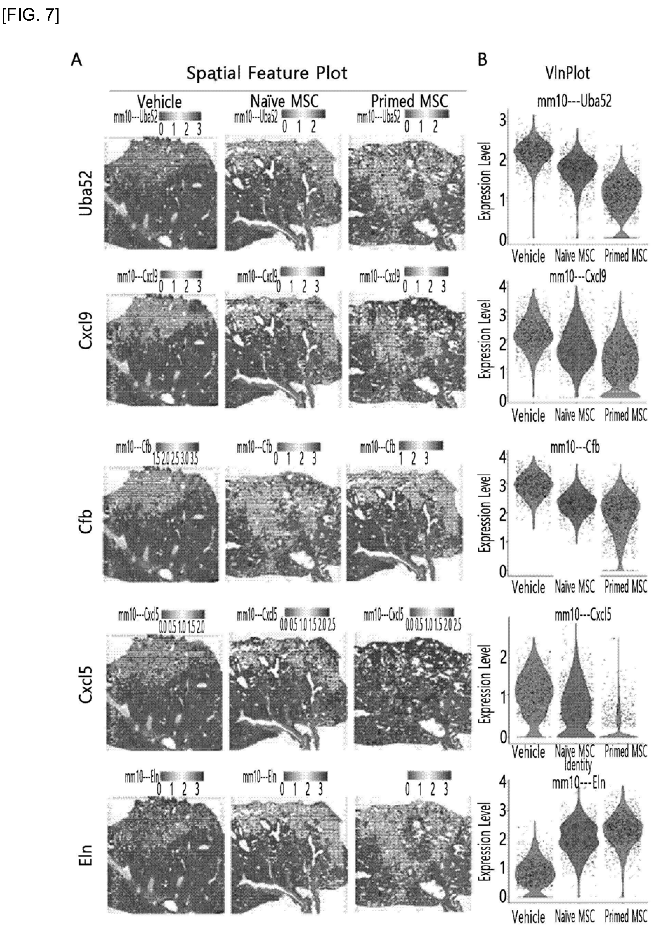

FIG. 7 shows the results of identifying the major factors of pure MSCs and priming MSCs for the top 5 inflammation-related genes with increased expression in acutely injured lungs. Specifically, (A) is a result showing that the expression of the top 5 genes decreased in both pure MSCs and priming MSCs through spatial characteristic distribution analysis, and (B) is a result showing that the expression levels of the top 5 genes decreased due to the influence of pure MSCs and priming MSCs in VlnPlot analysis.

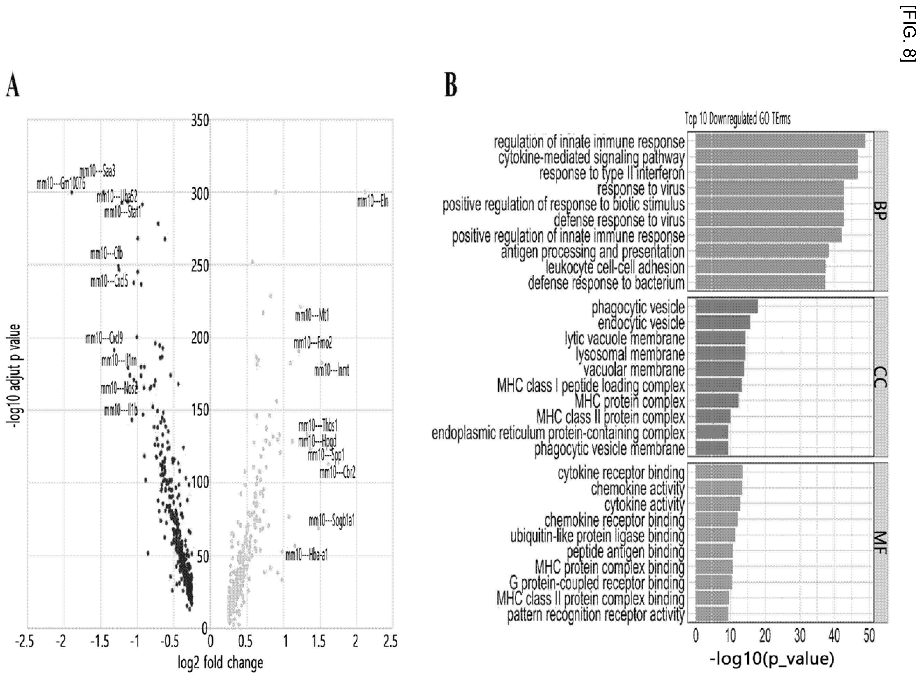

FIG. 8 is a figure showing the immunomodulatory effect of co-treated priming MSC prepared according to one embodiment of the present invention. Specifically, (A) is a result showing factors with reduced expression on the left and factors with increased expression on the right compared to the control group and priming MSCs, and (B) is a bar plot showing the reduced expression of the top 10 gene ontology terms for biological processes (BP), cell composition (CC), and molecular functions (MF).

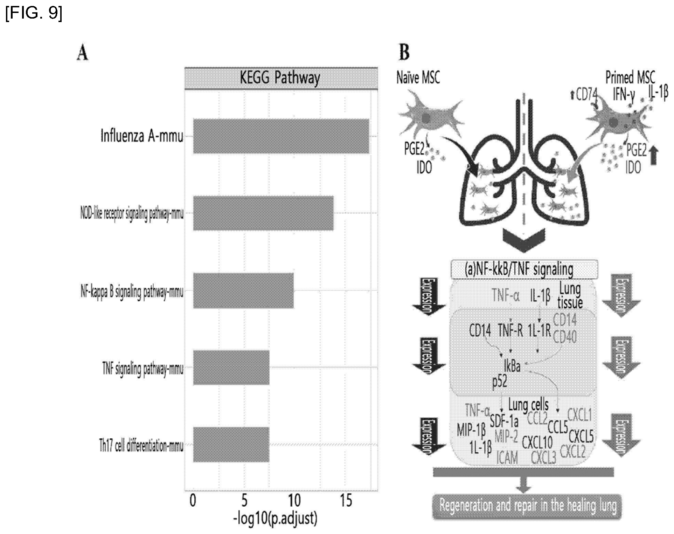

FIG. 9 is a figure showing the results of a treatment mechanism through spatial transcriptome analysis. Specifically, (A) is the analysis results of the reduction in KEGG pathway expression of ARDS-related genes, and (B) is the result showing the reduction of inflammatory factors by pure MSC and priming MSC in the NF-κB and TNF signaling pathways.

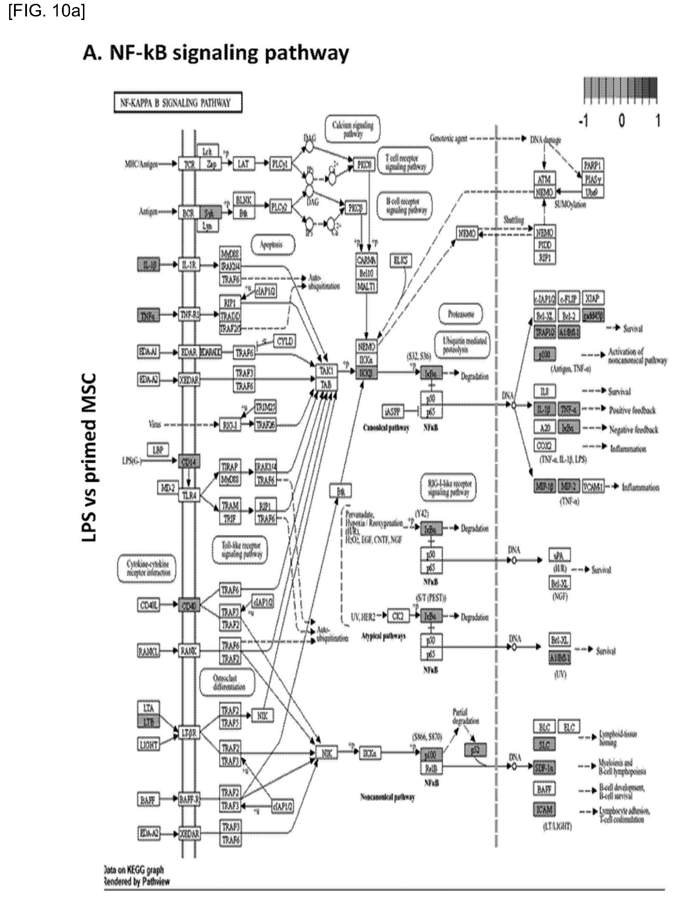

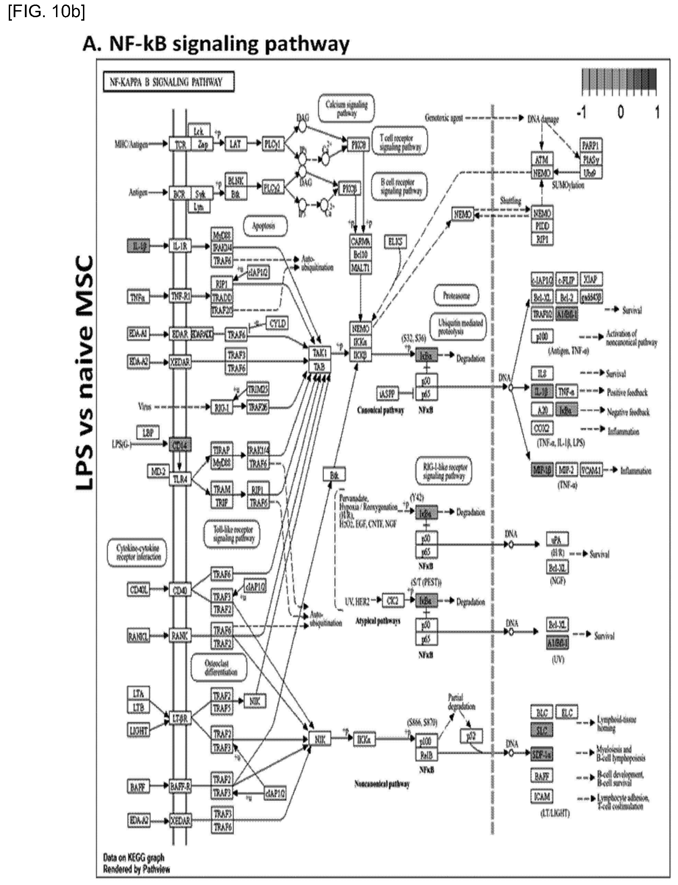

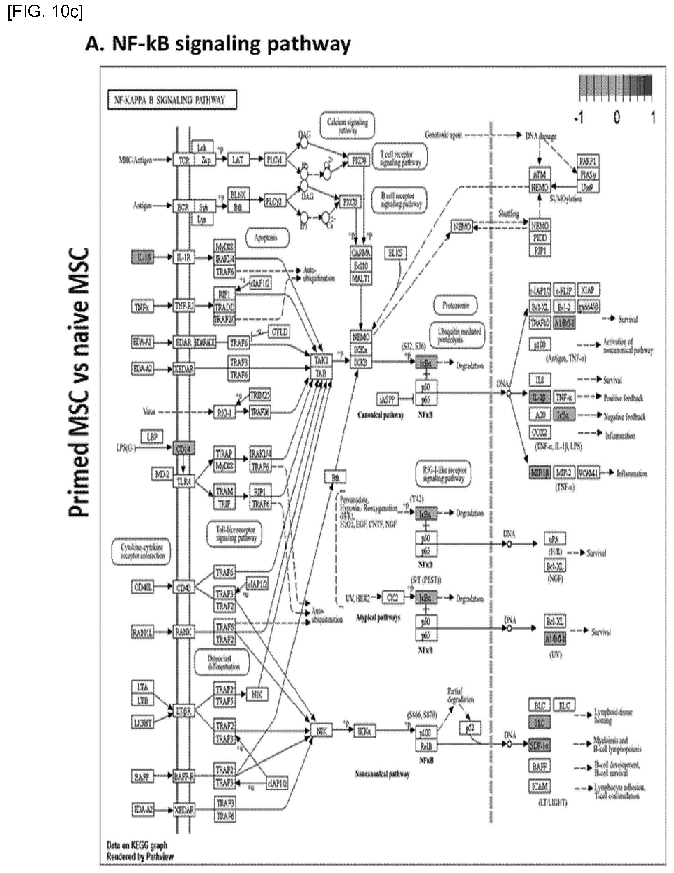

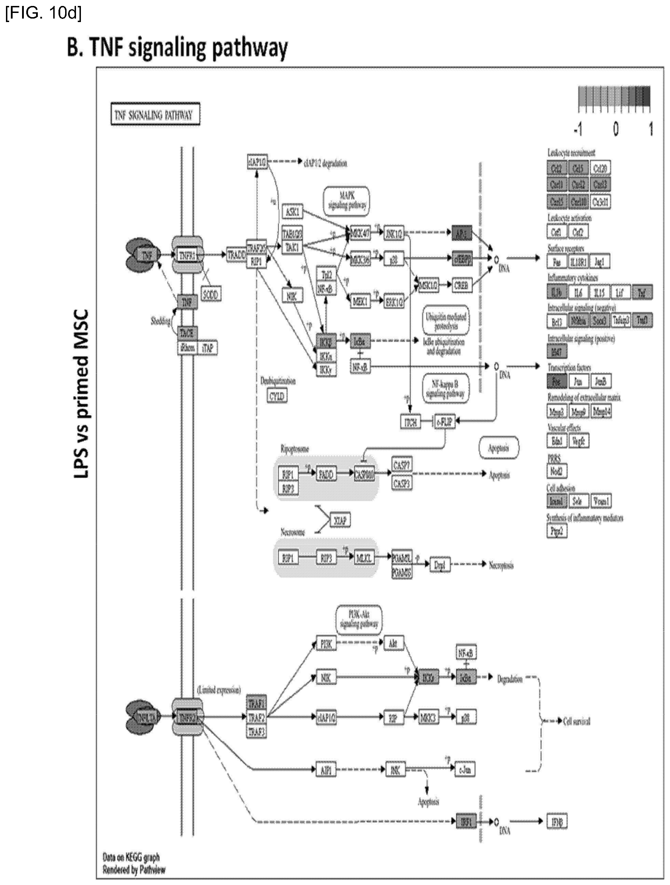

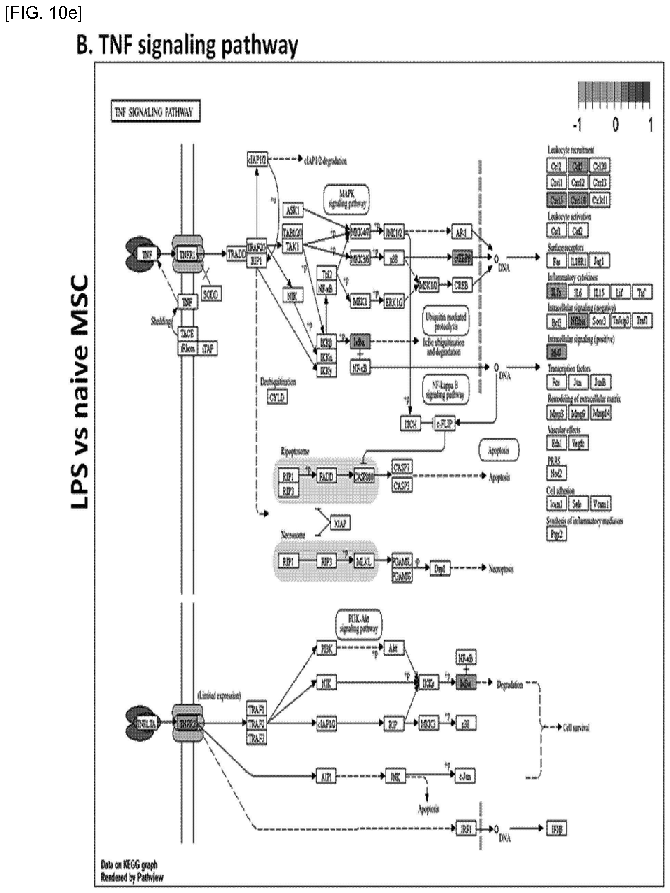

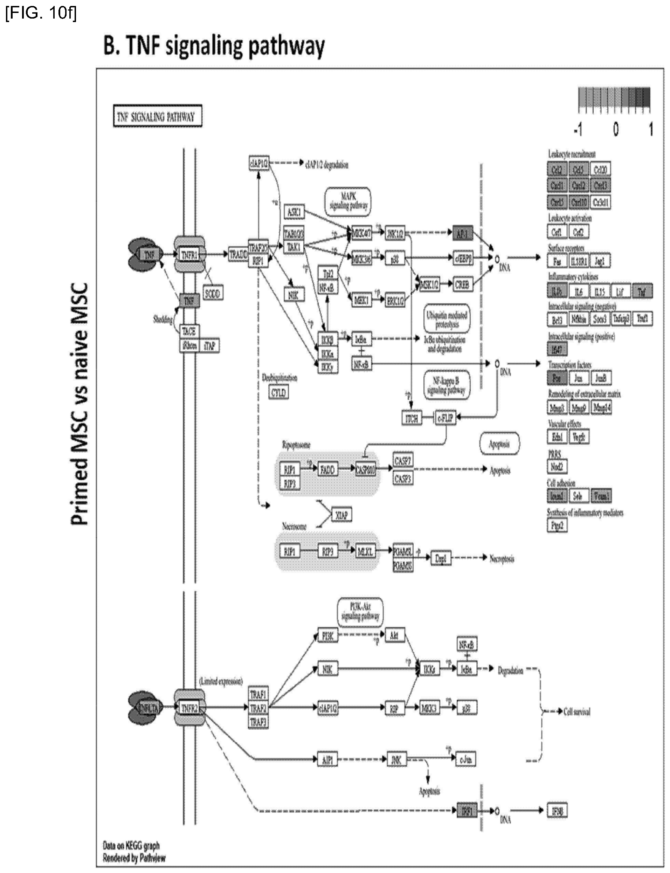

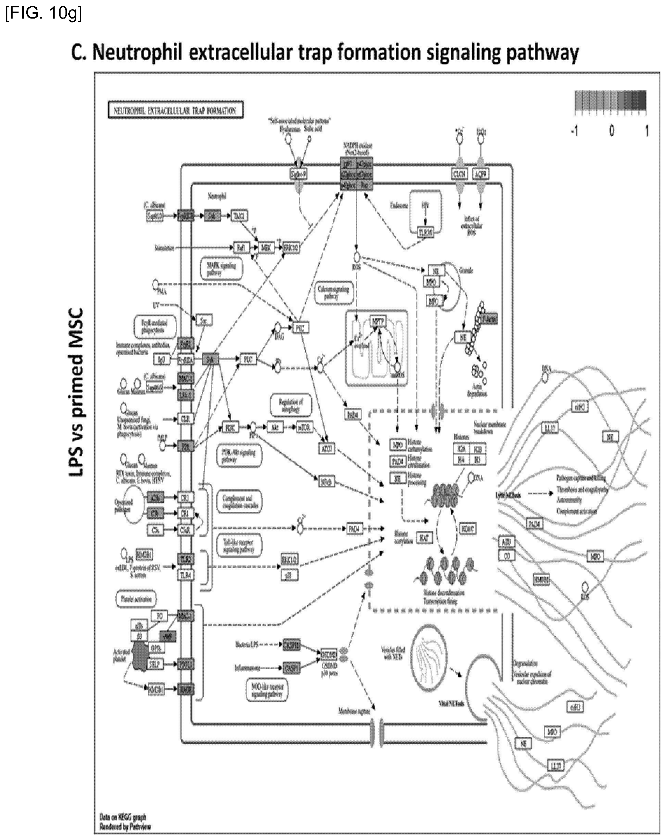

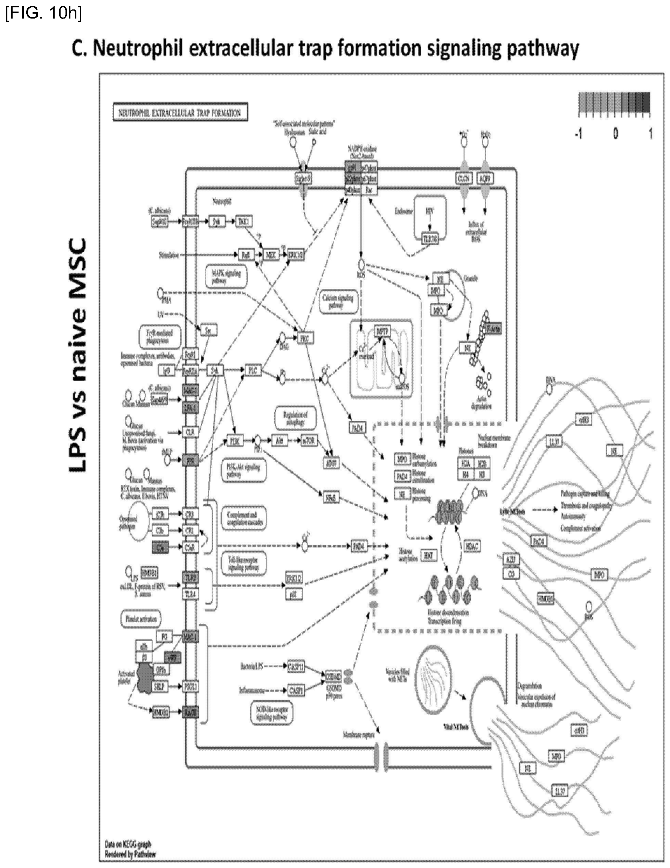

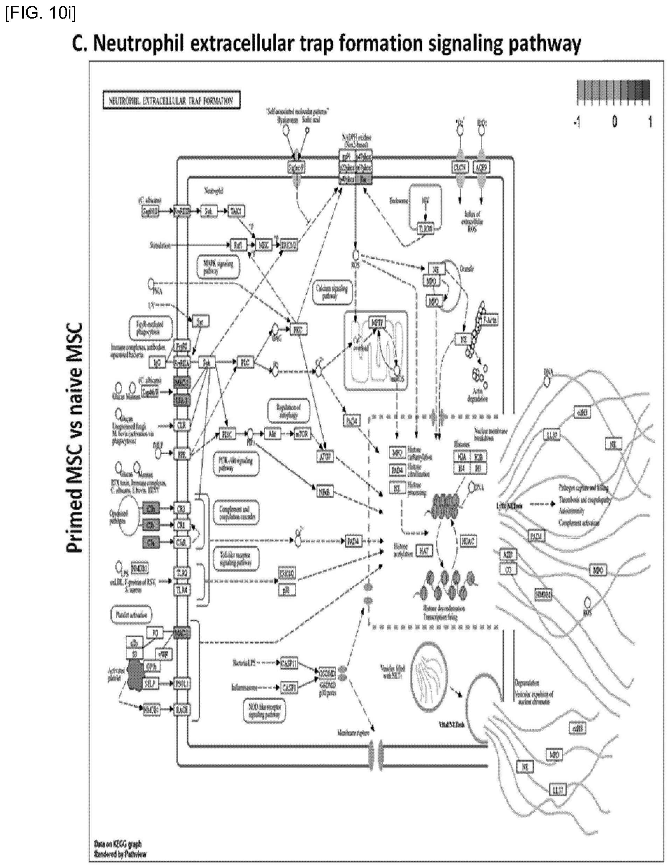

FIG. 10 shows the results of the therapeutic mechanism of pure MSC and co-treated priming MSC prepared according to one embodiment of the present invention in the analysis of the KEGG pathway for inflammation-related genes. Specifically, (A) is a diagram showing that pure MSCs and priming MSCs reduce inflammatory factors in the NF-κB signaling pathway, and that the priming MSCs show enhanced efficacy, (B) is a diagram showing that priming MSCs inhibit more inflammatory factors than pure MSCs in the TNF signaling pathway, and (C) is a diagram showing that pure MSCs and priming MSCs reduce inflammatory factors in the NET formation signaling pathway, and that priming MSCs show a more enhanced effect than pure MSCs.

### [Detailed Description of Preferred Embodiments]

The present invention will be described in detail as follows. Meanwhile, each description and embodiment described herein may be applied to other descriptions and embodiments, respectively. That is, all combinations of various elements described herein fall within the scope of the present invention. Further, the scope of the present invention is not limited by the specific description described below.

One aspect of the present invention provides a composition for enhancing immunomodulatory ability of a priming mesenchymal stem cell, comprising IL-1β, IFN-γ, or a combination thereof as an active ingredient.

In one embodiment, the mesenchymal stem cell is an umbilical cord blood-derived mesenchymal stem cell.

In a composition for enhancing immunomodulatory ability of a priming mesenchymal stem cell according to a foregoing embodiment, the umbilical cord blood-derived mesenchymal stem cell is derived from a human.

In a composition for enhancing immunomodulatory ability of a priming mesenchymal stem cell according to a foregoing embodiment, the IL-1β and IFN-γ are comprised at concentrations of 2 ng/mL to 50 ng/mL and 10 ng/mL to 50 ng/mL, respectively.

In a composition for enhancing immunomodulatory ability of a priming mesenchymal stem cell according to a foregoing embodiment, the IL-1β and IFN-γ are treated for less than 48 hours.

In a composition for enhancing immunomodulatory ability of a priming mesenchymal stem cell according to a foregoing embodiment, the IL-1β, IFN-γ, or a combination thereof enhances the immunomodulatory ability of the priming mesenchymal stem cell relative to that before treatment.

In a composition for enhancing immunomodulatory ability of a priming mesenchymal stem cell according to a foregoing embodiment, the immunomodulatory ability is immunosuppressive but not immunogenic.

In a composition for enhancing immunomodulatory ability of a priming mesenchymal stem cell according to a foregoing embodiment, the priming mesenchymal stem cell suppresses TNF-α secretion and promotes IL-10 secretion in a macrophage.

In a composition for enhancing immunomodulatory ability of a priming mesenchymal stem cell according to a foregoing embodiment, the priming mesenchymal stem cell suppresses secretion of TNF-α to a range of 3,000 pg/mL to 4,500 pg/mL and promotes secretion of IL-10 to a range of 400 pg/mL to 700 pg/mL in a macrophage.

In a composition for enhancing immunomodulatory ability of a priming mesenchymal stem cell according to a foregoing embodiment, the priming mesenchymal stem cell increases expression of an inflammation or immunoregulation-related gene.

In a composition for enhancing immunomodulatory ability of a priming mesenchymal stem cell according to a foregoing embodiment, the inflammation-related gene comprises one or more selected from CCL5, CCL2, SLAMF8, RSAD2, IDO1, CD74, HLA-DRB, and a combination thereof.

The immunoregulation-related gene comprises one or more of CXCL10, CXCL9, CXCL11, CXCL2, CCL20, SERPINB7, IDO1, PTGES, and a combination thereof.

Another aspect of the present invention provides a pharmaceutical composition for preventing or treating ARDS, comprising the composition for enhancing immunomodulatory ability of a priming mesenchymal stem cell, the composition comprising IL-1β, IFN-γ, or a combination thereof as an active ingredient.

In one embodiment, the composition increases elastin to regenerate tissue or lung damage resulting from ARDS.

In a pharmaceutical composition according to a foregoing embodiment, the composition increases serine protease inhibitor (SERPIN) superfamily gene expression to inhibit tissue and cellular damage resulting from neutrophil infiltration.

In a pharmaceutical composition according to a foregoing embodiment, the SERPIN superfamily gene is SERPINB2, SERPINB7, SERPING1, or SERPINA9.

In a pharmaceutical composition according to a foregoing embodiment, the composition further comprises a pharmaceutically acceptable carrier.

In a pharmaceutical composition according to a foregoing embodiment, the composition further comprises an excipient or an additive.

Still another aspect of the present invention provides a method for preventing or treating ARDS, comprising administering the pharmaceutical composition to an individual other than a human.

Still another aspect of the present invention provides a composition for preparing a priming mesenchymal stem cell having enhanced immunomodulatory ability, comprising IL-1β, IFN-γ, or a combination thereof as an active ingredient.

Still another aspect of the present invention provides a method for preparing a priming mesenchymal stem cell having enhanced immunomodulatory ability, comprising treating IL-1β, IFN-γ, or a combination thereof to a mesenchymal stem cell.

Still another aspect of the present invention provides a priming mesenchymal stem cell having enhanced immunomodulatory ability prepared by the method.

Still another aspect of the present invention provides a pharmaceutical composition for preventing or treating ARDS, comprising the priming mesenchymal stem cell as an active ingredient.

The present invention will be described in detail as follows. Meanwhile, each description and embodiment described herein may be applied to other descriptions and embodiments, respectively. That is, all combinations of various elements described herein fall within the scope of the present invention. Further, the scope of the present invention is not limited by the specific description described below.

Furthermore, those skilled in the art may recognize or identify numerous equivalents to the specific embodiments of the present invention described herein by using routine experimentation. Moreover, such equivalents are intended to be included within the present invention.

Additionally, a number of papers and patent documents have been cited throughout the present specification. The content of the cited papers and patent documents is incorporated herein by reference in their entirety to describe the level of the technical field to which the present invention belongs and the contents of the present invention more clearly.

The present invention is based on the project for the development of an ARDS therapy using stem cells specialized in immunomodulation under the pan-governmental regenerative medicine technology development project managed by the Korea Health Industry Development Institute of the Ministry of Health and Welfare (Unique No.: 1465038534/ Project No.: HH21C0021000023).

As used herein, a "stem cell" is a cell capable of differentiating into various tissues, i.e., an undifferentiated cell. "Priming stem cell" refers to a stem cell manipulated to enhance specific functions through a priming method, which increases therapeutic effects by applying specific stimuli, such as cytokines, hypoxic conditions, or chemical stimulation, to the stem cell, thereby providing a stem cell with therapeutic properties superior to those of pure MSCs.

In the present invention, a "stem cell that has not been treated for stimulation" may be used interchangeably with "pure stem cell", and a stem cell treated for stimulation such as cytokines is defined as a "priming stem cell".

In the present invention, the priming stem cell may be a stem cell treated with IL-1β, IFN-γ, or a combination thereof. Specifically, a stem cell treated with a combination of IL-1β and IFN-γ can be referred to as a priming stem cell, and can be used interchangeably with "IL-1β/IFN-γ priming stem cell" and "co-treated stem cell".

The stem cell may be of human or animal origin and may be derived from the umbilical cord, umbilical cord blood, bone marrow, fat, muscle, nerve, skin, amniotic membrane, or placenta. In one example, it may be a mesenchymal stem cell.

"Mesenchymal stem cell" refers to a heterogeneous population of stem cells capable of self-renewal and differentiation into other embryonic lineages, such as the mesoderm, endoderm, and ectoderm lineages. "Mesenchymal stem cell" may be used to mean a multipotent undifferentiated cell and is an adult stem cell capable of differentiating into various mesodermal cells, including adipocytes, osteoblasts, chondrocytes, cardiac cells, or muscle cells, or ectodermal cells such as neurons.

Specifically, the stem cell of the present invention may be an umbilical cord blood-derived mesenchymal stem cell.

As used herein, "interleukin 1 beta (IL-1β)" is one of the inflammatory cytokines that is also known as leukocyte pyrogen, leukocyte endogenous mediator, monocyte factor, lymphocyte activating factor, and other names, and is a cytokine protein encoded by the IL1B gene in humans. Interleukin-1 (IL-1) has two genes, IL-1α and IL-1β (the corresponding gene), and it is known that the IL-1β precursor is cleaved by cytoplasmic caspase 1 (interleukin-1 beta-converter) to form mature IL-1β.

As used herein, "interferon gamma (IFN-γ)" is a soluble cytokine composed of a dimer and is the sole component of type 2 interferon. The IFN-γ, formerly known as 'immune interferon', was first discovered as a product of human leukocytes stimulated by phytohemagglutinin. In humans, the IFN-γ protein is reported to be encoded by the *IFNG* gene.

With respect to the IL-1β, IFN-γ, or combination thereof, IL-1β may be comprised at a concentration of 2 ng/mL to 50 ng/mL and IFN-γ at a concentration of 10 ng to 50 ng.

The IL-1β, IFN-γ, or a combination thereof may be treated for less than 48 hours. Specifically, it can be treated for more than 0 hours and less than 48 hours, 0.1 hours to less than 48 hours, or 0.5 hours to less than 48 hours.

The IL-1β, IFN-γ, or a combination thereof may enhance immunomodulatory ability of the priming mesenchymal stem cell relative to that before treatment.

The immunomodulatory ability means being immunosuppressive but not immunogenic.

The priming mesenchymal stem cell may suppress TNF-α secretion and promote IL-10 secretion in a macrophage.

As used herein, the term "macrophage" refers to a cell that plays a role in inducing inflammation and tissue destruction by secreting inflammatory cytokines and tissue-degrading enzymes. Among macrophages, "M0 macrophages" are undifferentiated macrophages, *i.e*., macrophages that have not yet differentiated, and differentiate or polarize into anti-inflammatory M2 macrophages and pro-inflammatory M1 macrophages. Among the above, an M1 macrophage is generally activated by IFN-γ or lipopolysaccharide (LPS), produces pro-inflammatory cytokines (TNF-α, IL-1β, IL-6, *etc.*), eliminates a microorganism through phagocytosis, and initiates an immune response. Meanwhile, an M2 macrophage is differentiated and activated by specific cytokines such as IL-4 and IL-13, and secretes various anti-inflammatory cytokines such as TGF-α and IL-10.

Whether an M0 macrophage differentiates into an M1 macrophage or whether differentiation is inhibited can be confirmed by measuring the production amount or secretion amount of the anti-inflammatory cytokine TNF-α. Whether an M0 macrophage differentiates into an M2 macrophage or whether differentiation is promoted can be confirmed by measuring the secretion amount or production amount of IL-10 secreted during M2 macrophage differentiation.

Therefore, if the priming mesenchymal stem cell inhibits the secretion of TNF-α and promotes the secretion of IL-10 within a macrophage, it means that differentiation into an M1 macrophage is inhibited, and the differentiation of an M0 macrophage into an M2 macrophage is promoted. That is, a primed mesenchymal stem cell can effectively inhibit the differentiation of an M0 macrophage into an M1 macrophage by significantly suppressing TNF-α secretion compared to a pure MSC, and effectively promote differentiation of an M0 macrophage into an M2 macrophage by increasing IL-10 secretion.

As used herein, the term "differentiation" refers to the phenomenon in which the structure or function of cells becomes specialized during growth through cell division and proliferation, *i.e*., the change in form or function of each biological cell, tissue, *etc.*, of a microorganism in order to perform the tasks assigned to each. For example, differentiation refers to the emergence of qualitative differences between parts of a biological system that were initially homogeneous in ontogenesis, or the state in which it is divided into qualitatively distinguishable subsystems as a result.

The term "differentiation inhibition" refers to slowing down and/or reducing the growth of cells through division and proliferation compared to normal differentiation levels, and the term "differentiation promotion" refers to enabling differentiation to occur faster and/or more frequently than normal differentiation levels.

The "differentiated cell" refers to any cell that is in the process of differentiating into a somatic lineage or has finally differentiated. That is, it refers to a cell that constitutes an adult body and has limited differentiation and self-production capabilities.

The stem cell culture medium of the present invention may use a medium known for stem cell culturing, and specifically, KSB-3 medium may be used.

The present invention may inhibit the secretion of TNF-α to a range of 3,000 pg/mL to 4,500 pg/mL and promote the secretion of IL-10 to a range of 400 pg/mL to 700 pg/mL in a macrophage.

In the present invention, the priming mesenchymal stem cell may increase the expression of an inflammation or immunoregulation-related gene.

The inflammation-related gene comprises one or more selected from CCL5, CCL2, SLAMF8, RSAD2, IDO1, CD74, HLA-DRB, and a combination thereof, and the immunoregulation-related gene comprises one or more of CXCL10, CXCL9, CXCL11, CXCL2, CCL20, SERPINB7, IDO1, PTGES, or a combination thereof.

In a specific embodiment of the present invention, it was confirmed that treatment of a mesenchymal stem cell with IL-1β, IFN-γ, or a combined treatment thereof inhibits T cell proliferation and effectively promotes M2 macrophage activation, and inhibits TNF-α secretion and promotes IL-10 secretion within a macrophage, thereby effectively enhancing immunomodulatory ability in a primed umbilical cord blood-derived mesenchymal stem cell. In addition, it was confirmed that IL-1β, IFN-γ, or a combined treatment thereof increased the gene expression of CXCL1, CXCL2, CXCL3, CXCL5, CXCL6, and CXCL8, which play a role in neutrophil induction, and CXCL9, CXCL10, and CXCL11, which play a role in T cell induction, and increased the expression of CCL2 and CCL5, which promote monocyte and macrophage induction, and CCL20, which promotes lymphocyte induction, and that the expression of all CXCL and CCL family genes except CCL5 increased when a combined treatment of IFN-γ and IL-1β was compared to IFN-γ alone.

In the present invention, the pharmaceutical composition for preventing or treating ARDS, comprising IL-1β, IFN-γ, or a combination thereof as an active ingredient, may be applied as a pharmaceutical composition for preventing or treating acute ARDS.

In addition, the pharmaceutical composition may be applied in a method for preventing or treating ARDS by administering the pharmaceutical composition to an individual.

In particular, the above pharmaceutical composition can be applied as a cell therapy agent.

The terms, "IL-1β", "IFN-γ", "priming mesenchymal stem cell", "immunomodulatory ability", *etc.*, are as described above.

As used herein, "acute respiratory distress syndrome (ARDS)" refers to a disease that occurs mainly due to infection, trauma, or other systemic inflammatory response, and major characteristics thereof may be life-threatening respiratory failure due to widespread lung inflammation, with an acute onset of inflammation and increased permeability of the alveolar-capillary barrier leading to respiratory dysfunction.

The term, "cell therapeutics" refers to a treatment involving direct injection of a living cell into a patient, and is known as "cell therapeutics". Cell therapeutics refers to the living cell used in cell therapy. The Regulation on Safety of Pharmaceuticals, *etc.* defines cell therapeutics as pharmaceuticals manufactured by manipulating a living autologous cell, allogeneic cell, or xenogeneic cell through physical, chemical, or biological methods, such as culturing, proliferating, or selecting the cell *in vitro.* However, this excludes cases where a physician performs only the minimum manipulations that do not compromise safety (simple separation, washing, freezing, thawing, *etc*., within the range where biological characteristics are maintained) on an autologous or allogeneic cell during the relevant surgery or treatment process within a medical institution.

The pharmaceutical composition may increase serine protease inhibitor (SERPIN) superfamily gene expression to inhibit tissue and cellular damage resulting from neutrophil infiltration.

The term "serine protease inhibitor (serpin) superfamily gene" refers to a gene that plays an important role in regulating proteases to prevent tissue damage and the premature death of immune cells. Specifically, it may be SERPINB2, SERPINB7, SERPING1, or SERPINA9, and more specifically, SERPINB2 or SERPINB7.

As used herein, the term "prevention" refers to any activity inhibiting or delaying the onset of ARDS by administering the composition, and the term "treatment" refers to any activity that improves or beneficially changes the symptom of ARDS by administering the composition.

The pharmaceutical composition of the present invention may further comprise a pharmaceutically acceptable carrier.

The "pharmaceutically acceptable carrier" may refer to a carrier or diluent that does not stimulate living organisms and does not impair the biological activity and properties of the compound to be injected. The types of carrier that may be used in the present invention are not particularly limited, and any pharmaceutically acceptable carrier conventionally used in the relevant technical field may be used. Non-limiting examples of the carrier include saline solution, sterile water, Ringer's solution, buffered saline solution, albumin injection solution, dextrose solution, maltodextrin solution, glycerol, and ethanol. These examples may be used alone or in combination of two or more. In addition, the pharmaceutical composition may be formulated into injection forms such as solutions, suspensions, or emulsions, as well as capsules, granules, or tablets, by further adding diluents, dispersing agents, surfactants, binders, or lubricants.

The pharmaceutical composition may have any one formulation selected from the group consisting of tablets, pills, powders, granules, capsules, suspensions, oral liquid preparations, emulsions, syrups, sterile aqueous solutions, non-aqueous solvents, suspensions, emulsions, lyophilized preparations, and suppositories, and may have various formulations for oral or parenteral use. When formulated, the pharmaceutical composition is prepared using diluents or excipients conventionally used in the art, such as fillers, extenders, binders, wetting agents, disintegrants, and surfactants. Solid preparations for oral administration include tablets, pills, powders, granules, capsules, *etc.* , and these solid preparations are prepared by mixing at least one or more excipients, such as starch, calcium carbonate, sucrose, lactose, and gelatin, with one or more compounds. Further, in addition to simple excipients, lubricants such as magnesium stearate and talc may be used. Examples of liquid preparations for oral administration correspond to suspensions, solutions, emulsions, syrups, *etc.,* and in addition to commonly used simple diluents such as water and liquid paraffin, various excipients such as wetting agents, sweeteners, flavoring agents, and preservatives may be included. Preparations for non-oral administration include sterilized aqueous solutions, non-aqueous solvents, suspensions, emulsions, lyophilized preparations, and suppositories. As non-aqueous solvents and suspending agents, propylene glycol, polyethylene glycol, vegetable oils such as olive oil, injectable esters such as ethyl oleate, etc. may be used. As bases for suppositories, Witepsol, Macrogol, Tween 61, cocoa butter, lauric acid, glycerogelatin, etc. may be used.

In addition, the pharmaceutical composition of the present invention may be administered in a pharmaceutically effective amount.

As used herein, the term "pharmaceutically effective amount" refers to an amount that is sufficient to treat a disease at a reasonable benefit/risk ratio applicable to medical treatment, and the level of the effective dose may be determined depending on factors including the individual type, severity of disease, age, sex, type of disease, activity of the drug, drug sensitivity, time of administration, route of administration, excretion rate, duration of treatment, and concurrently used drugs, as well as other factors well known in the medical field. The composition of the present invention may be administered as a stand-alone treatment or in combination with other treatments, and may be administered sequentially or simultaneously with conventional treatments. Additionally, it may be administered as a single dose or multiple doses. It is important to administer the minimal effective amount that maximizes efficacy without causing side effects, while taking all of the above elements into account, and the amount may be readily determined by those skilled in the art.

In addition, the pharmaceutical composition may be administered orally or parenterally (*e.g*., intravenously, subcutaneously, intraperitoneally, or topically) depending on the desired method. In one example, it may be administered parenterally.

The pharmaceutical composition of the present invention may be administered as a stand-alone therapeutic agent or together with other active ingredients that exhibit a preventive or therapeutic effect for ARDS. When the pharmaceutical composition of the present invention is administered together with other active ingredients, this may be referred to as "combined administration" or "complex administration". In particular, the antibody or its antigen-binding fragment effective for treating ARDS according to the present invention and other active ingredients may be administered as a single mixture or in separate forms.

In the present invention, "combined administration", "administered in combination", or "co-administered" shall be understood to indicate simultaneous, individual, sequential, or reverse administration, and the order thereof shall be understood as unlimited. That is, co-administration is not limited merely to simultaneous administration but may be a form of administration that acts together on the individual to enable each substance to perform its inherent function at a level equivalent to or greater than its original function. Accordingly, when the term "co-administration" is used in the present invention, the administration is not particularly limited as long as it is sequential, reverse, or individual, the order of administration, and the interval between the administration of the second component can be adjusted so as not to lose the beneficial effects of the co-administration.

As used herein, the term "individual" may be used interchangeably with "subject" and refers to mammals including cattle, dogs, pigs, chickens, sheep, horses, and humans, but is not limited thereto.

In the present invention, the IL-1β, IFN-γ, or a combination thereof may be applied as the composition for preparing a priming mesenchymal stem cell having enhanced immunomodulatory ability, comprising IL-1β, IFN-γ, or a combination thereof as an active ingredient.

In addition, it may be applied to a method for preparing a priming mesenchymal stem cell having enhanced immunomodulatory ability, comprising treating IL-1β, IFN-γ, or a combination thereof to a mesenchymal stem cell.

In addition, it may be applied to a priming mesenchymal stem cell having enhanced immunomodulatory ability prepared by the method.

In addition, it may be applied to a pharmaceutical composition for preventing or treating ARDS, comprising the priming mesenchymal stem cell prepared by the method as an active ingredient.

The terms, "IL-1β", "IFN-γ", "priming mesenchymal stem cell", "immunomodulatory ability", and the like are as described above.

### [Mode for Carrying Out the Invention]

The present disclosure will be described in detail by way of Examples. The Examples are provided to specifically explain the present invention, and the scope of the present invention is not limited by the Examples.

### Experimental Example 1: hUCB-MSC culturing

hUCB-MSCs were obtained from Kangstem Biotech GMP Center (South Korea) and cultured in KSB-3 basal medium (Kangstem Biotech, South Korea) supplemented with 10% fetal bovine serum (FBS; Gibco, USA) and 100 µg/mL primosin (Invitrogen, USA). In the present experiment, hUCB-MSCs cultured for 8 consecutive passages were used. The MSCs were primed with interferon gamma (IFN-γ; Peprotech, USA) or interleukin-1 beta (IL-1β; Peprotech, USA) for 24 hours to enhance immunomodulatory function or increase responsiveness in an inflammatory environment. The priming MSCs were cultured for 24 hours under the following three conditions: (1) culturing with 20 ng/mL IFN-γ, (2) culturing with 5 ng/mL IL-1β, and (3) culturing with 20 ng/mL IFN-γ and 5 ng/mL IL-1β. All cells were maintained at 37°C in a 5% CO₂ incubator.

### Experimental Example 2: Mixed lymphocyte reaction (MLR analysis)

To confirm the immunogenicity and immunosuppressive ability of the hUCB-MSCs, direct and indirect co-culture methods were performed according to the 'Mesenchymal Stem Cell Therapy Mixed Lymphocyte Response Test' established by the MFDS.

### Experimental Example 2-1: Direct Co-Culture Assay

To confirm the immunogenicity of the priming MSCs prepared in Example 1 above, peripheral blood mononuclear cells (PBMC) activated with concanavalin A (ConA; Invitrogen, USA) were cultured with MSCs treated with mitomycin C (MMC; Sigma-Aldrich, USA). Specifically, 1 x 10⁵ PBMCs were directly co-cultured with 1 x 10⁴ MSCs in a 10:1 ratio in a 96-well plate for 96 hours.

### Experimental Example 2-2: Indirect Co-Culture Assay

To confirm the immunosuppressive ability of the priming MSCs prepared in Example 1 above, PBMCs (1 x 10⁶ cells) activated with ConA were cultured at the bottom of a 24-well plate, and priming hUCB-MSCs (1.0 x 10⁵ cells) were placed in the top insert so that the ratio of PBMCs to MSCs was 10:1. Subsequently, co-culture was carried out for 96 hours, and PBMC proliferation was confirmed by bromodeoxyuridine or 5-bromo-2'-deoxyuridine (BrdU; Roche, Switzerland) insertion assay.

### Experimental Example 3: M1 and M2 macrophages

Human monocytes (THP-1 (TIB-202); ATCC, USA) were incubated in RPMI-1640 medium (ATCC, USA) containing 10% FBS, 50 pM β-mercaptoethanol (β-MET, Gibco, USA), and 100 µg/mL primosin at 37°C in a 5% CO₂ incubator. The cultured THP-1 monocytes were seeded at a density of 1 x 10⁶ cells per well of a 6-well plate, and differentiated into M0 macrophages by adding 150 nM phorbol 12-myristate 13-acetate (PMA; Sigma, USA) and 10% FBS to RPMI medium and incubating for 24 hours. Subsequently, 20 ng/mL IFN-γ, 100 ng/mL LPS (Sigma, #8630), and 10% FBS were added to the RPMI medium to polarize the macrophages into M1 macrophages. Thereafter, hUCB-MSC (1 x 10⁶ cells) were inoculated into a transwell and co-cultured for 72 hours. Then, the concentration of TNF-α in the culture medium was measured using the Human TNF-α Quantikine ELISA Kit (R&D Systems, USA) according to the manufacturer's instructions. Additionally, to confirm the ability of hUCB-MSCs to induce M2 differentiation and IL-10 secretion, THP-1 monocytes were cultured in RPMI medium supplemented with 10% FBS and 150 nM PMA for 24 hours, and then differentiated into M2 macrophages by adding 150 nM PMA and culturing for 24 hours. Thereafter, hUCB-MSC (1 x 10⁶ cells) were inoculated into a transwell and co-cultured for 72 hours. Then, the concentration of IL-10 was measured using the Human IL-10 Quantikine ELISA Kit (R&D Systems, USA) according to the manufacturer's instructions.

### Experimental Example 4: RNA sequencing

For RNA sequencing (RNA-seq), whole RNA samples were isolated from pure MSCs and IFN-γ/IL-1β priming MSCs using the Truseq stranded mRNA library prep kit (Illumina Inc., USA). Then, the amount and quality of the isolated RNAs were measured using a Nanodrop device (Thermo Scientific, USA). mRNA-seq raw data were used to estimate expression levels using Kallisto, and differential expression analysis was performed using edgeR of the R package to classify differentially expressed genes (DEGs) into up-regulated (Case/Control): FDR (adjusted p-value) < 0.05 and log2FoldChange ≥ 1, and down-regulated (Case/Control): FDR < 0.05 and log2FoldChange ≤ -1. Subsequently, using the ontology database (org.Hs.eg.db) of the R package, gene ontology (GO) classification and functional distribution analysis were performed on genes that differed by more than two-fold and had FDR < 0.05. The Pathview of the KEGGREST package and the R package were used for the analysis of the Kyoto Encyclopedia of Genes (KEGG) and Genomes. Gene set enrichment analysis (GSEA) was performed using the GSEA 4.3.3 tool and MSigDB (core gene set and C5 ontology gene set).

### Experimental Example 5: Experimental animals and drugs

The LPS-induced mouse model is an animal model widely used in ARDS research. To this end, a sterile LPS solution (Sigma-Aldrich, *E. coli* 0127) was dissolved in phosphate-buffered physiological saline (PBS) to prepare a solution at a concentration of 5 mg/kg. The experiment was conducted using 8-week-old male C57BL/6 mice (Koatech, Korea) divided into four groups: normal group (NC, n=4), control group (LPS-induced ARDS, n=14), pure MSC group (pure MSC administration after LPS-induced ARDS, n=14), and IFN-γ/IL-1β priming MSC group (priming MSC administration after LPS-induced ARDS, n=14). All mice used here were anesthetized with alfaxalone (Alfaxan^{®} , Jurox, USA) and xylazine (Rompun^{®}, Bayer AG, Germany).

In the ARDS model, lung injury was induced by administering LPS into the trachea at a dose of 5 mg/kg. In the experimental group, pure MSC or priming MSC was injected into the tail vein at a dose of 1×10 cells/200 µL. In the control group, PBS was injected intravenously. The intravenous injection was administered twice, at 4 hours and on Day 3 after LPS administration, and the mice were sacrificed on Day 4 after LPS administration.

### Experimental Example 6: Visual inspection

The degree of lung surface damage in the animal models prepared in Experimental Example 5 was visually confirmed. Specifically, only damage visible on the lung surface was evaluated, and scores were assigned according to partial minor, total minor, partial moderate, total moderate, partial severe, and total severe congestion. Scores were assigned from 0 to 6, reflecting the severity of the lesion in each category.

### Experimental Example 7: Histological analysis

After sacrificing the mice on Day 4 after LPS injury, the lungs were excised and fixed in formalin for histological analysis. The fixed lung was embedded in paraffin and then cut into pieces with a thickness of 5 µm. Afterwards, the lung pieces were stained with hematoxylin and eosin to determine the severity of lung damage. Severity was assessed using four pathological categories (alveolar congestion, hemorrhage, inflammation, and alveolar wall thickness), and a total score of 16 points was calculated by assigning a score from 0 to 4 points based on the severity of lesion in each category (see Table 1 below).

**[Table 1]**

| Score | Congestion | Hemorrhage | Inflammation | Thickness |
|---|---|---|---|---|
| 0 | None | | | |
| 1 | Minimal | | | |
| 2 | Mild | | | |
| 3 | Moderate | | | |
| 4 | Maximum | | | |
| SUM | Congestion + Hemorrhage + Inflammation + Thickness | | | |

### Experimental Example 8: BALF analysis

After inducing ARDS in mice with LPS, bronchoalveolar lavage fluid (BALF) was collected from the left lung lobe on Day 4. Specifically, BALF was centrifuged and the concentrations of TNF-α and IL-6 cytokines in the supernatant were identified (R&D Systems, USA).

### Experimental Example 9: Spatial transcriptome analysis

Lung tissue samples from the control group (LPS), pure MSC-treated group, or priming MSC-treated group were immediately frozen and included in the OCT compound. Subsequently, spatial transcriptome analysis was performed using the visium platform (10x Genomics, Inc., CA, USA). Data analysis was performed using programming languages such as R (version 4.3.1) and python (version 3.8.17) and pipelines including Seurat (version 4.4.0), Scanpy (version 1.9.4), Scanorama (version 1.7.3), and SpaceRanger (version 2.1.1).

GRCh38 (Homo sapiens) and mm10 (Mus musculus) were used as reference genomes. Normalization was performed by adjusting the count per spot for each sample using Scanpy. Additionally, cell type abundance analysis (xCell) was performed on 64 immune and stromal cell types.

### Experimental Example 10: Statistical analysis

All data were analyzed using GraphPad Prism version 5 (GraphPad software) and reported as mean ± standard error of mean (SEM). One-way ANOVA was used to compare multiple groups, and then Tukey post-hoc analysis was used for pairwise comparisons of the groups. Student t-test (two-tailed unpaired) was used to compare two values. In particular, p < 0.05 indicates a statistically significant difference (*, p < 0.05; **, p < 0.01; ***, p < 0.005).

### Example 1: Confirmation of the immunomodulatory ability of cord blood-derived priming mesenchymal stem cells treated with IL-1β, IFN-γ, or a combination thereof

### Example 1-1. Confirmation of the immune cell proliferation inhibitory effect and immunogenicity of priming MSCs and confirmation of suitable treatment concentration

Indirect co-culture was performed to confirm the immunosuppressive effect of hUCB-MSC. In the present analysis, the proliferation rate of PBMCs was measured for 72 hours through co-culture with pure MSCs or priming MSCs after activation with ConA.

As a result, as can be seen in FIG. 1A, both pure MSC and priming MSC were found to significantly inhibit the activation and proliferation of PBMC. Specifically, compared to the control group, the inhibition of PBMC proliferation was 83.4% in pure MSCs, 65.8% in IL-1β-priming MSCs, and 88.5% in IFN-γ-priming MSCs. In particular, it was confirmed that the inhibition was significantly lower at 60.8% in priming MSCs co-treated with IL-1β/IFN-γ, and was also confirmed to be significantly lower compared to priming MSCs treated with IL-1β or IFN-γ alone (FIG. 1A). This indicates that priming MSCs co-treated with IL-1β/IFN-γ showed the highest proliferation inhibitory effect, suggesting that they possess greater potential for regulating immune responses.

Subsequently, the immunogenicity of hUCB-MSC was confirmed.

First, to evaluate the potential for immunoinduction, a direct co-culture method was performed by co-culturing PBMCs with pure MSCs, MSCs primed with IFN-γ, MSCs priming with IL-1β, and MSCs priming in combination with IFN-γ and IL-1β.

As a result, as can be seen in FIG. 2, it was confirmed that no immunogenicity was observed in any of the MSC groups.

Next, to determine the appropriate treatment concentrations of IFN-γ and IL-1β, immunogenic cord blood-derived MSCs were treated with IFN-γ and IL-1β at different concentrations and times to examine changes in HLA-DR expression levels. For reference, the HLA-DR gene is one of the genes that can increase immunogenicity.

As a result, as can be seen in Table 2 below, it was confirmed that HLA-DR significantly increased compared to the control when IFN-γ was treated at a concentration of 20 ng/mL and IL-1β at a concentration of 5 ng/mL for more than 48 hours.

**[Table 2]**

| | IFN- 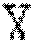 (ng/ml) | IL-1β (ng/ml) | HLA-DR(%) |
|---|---|---|---|
| Day1 | 0 | 0 | 1.09 |
| | 10 | 0 | 0.69 |
| | 20 | 0 | 0.91 |
| | 50 | 0 | 1.35 |
| | 100 | 0 | 1.72 |
| | 20 | 5 | 1.59 |
| | | | |
| Day2 | 0 | 0 | 0.12 |
| | 10 | 0 | 11.45 |
| | 20 | 0 | 10.36 |
| | 50 | 0 | 8.69 |
| | 100 | 0 | 9.03 |
| | 20 | 5 | 3.87 |
| | | | |
| Day4 | 20 | 0 | 33.9 |
| | 20 | 5 | 17.47 |

In addition, the conditions for inhibiting mixed lymphocytes were screened when MSCs were treated with CL429, MPLA, IL-1β, and IFN-g at different concentrations to further confirm the appropriate treatment concentration.

As a result, as can be seen in FIG. 3, it was confirmed that IFN-γ significantly inhibited mixed lymphocyte activity under all conditions except when the concentration was 2 ng/mL.

Synthesizing these results, it was found that the optimal treatment concentrations for IL-1β at a concentration of 2 ng/mL to 50 ng/mL and for IFN-γ at a concentration of 10 ng to 50 ng are those that simultaneously possess immunomodulatory ability without increasing immunogenicity, and with respect to the treatment time, it was found that immunogenicity does not increase when the two cytokines are treated for less than 48 hours.

### Example 1-2: Confirmation of TNF-α and IL-10 secretion ability

To confirm the anti-inflammatory effects of pure MSCs and priming MSCs on macrophages, the secretion of inflammatory cytokines from M1/M2 macrophages was measured.

First, the level of TNF-α secretion was checked in M1 macrophages stimulated with LPS/IFN-γ and co-cultured with hUCB-MSCs for 72 hours. As a result, as can be seen in FIG. 1B, the TNF-α concentration increased to 13,846 pg/mL with LPS/IFN-γ treatment, but it was confirmed that the TNF-α concentration decreased significantly when co-cultured with pure MSC, IL-1β-priming MSCs, IFN-γ-primig MSCs, or MSCs primed with a combination of IL-1β and IFN-γ (FIG. 1B). When quantified, the TNF-α secretion inhibition rate was found to be 3,300 pg/mL (23.9%) in pure MSCs, 4,270 pg/mL (30.8%) in IL-1β-priming MSCs, 3,191 pg/mL (23.0%) in IFN-γ-priming MSCs, and 2,193 pg/mL (15.8%) in IL-1β/IFN-γ combined-priming MSC, confirming that the inhibitory efficacy was synergistically reduced compared to pure MSCs or MSCs treated individually with IL-1β or IFN-γ.

This indicates that priming MSCs co-treated with IL-1β/IFN-γ are most effective in inhibiting TNF-α secretion.

Next, IL-10 secretion was measured in co-cultures of pure MSCs or priming MSCs with M0 macrophages.

As a result, as can be seen in FIG. 1C, it was confirmed that priming MSCs co-treated with IL-1β/IFN-γ induced IL-10 secretion levels at significantly higher levels compared to pure MSCs, IL-1β-priming MSCs, or IFN-γ-priming MSCs, respectively (FIG. 1C).

Based on these results, it was confirmed that priming MSCs treated with IL-1β or IFN-γ exhibited excellent immunomodulatory ability without inducing immunogenicity, and among them, priming MSCs treated with a combination of the two showed significantly superior immunomodulatory ability compared to priming MSCs with individual treatment.

### Example 2: Confirmation of molecular mechanisms and characterization of primed MSCs via RNA sequencing

In order to elucidate the molecular mechanism and characteristics of the immunosuppressive ability of the IL-1β/IFN-γ combined-priming MSC prepared in Example 1 above compared to pure MSC, total mRNA sequencing was performed and differentially expressed genes (DEG) analysis was conducted. In the present analysis, 499 up-regulated DEGs and 326 down-regulated DEGs were identified and visualized as a volcano plot (FIG. 4A).

As a result, as can be seen in FIG. 4A, the top 10 upregulated DEGs in priming MSCs co-treated with IL-1β/IFN-γ were identified as major immune system components involved in the regulation of inflammation and immune responses.

To understand the biological functions of upregulated DEGs, GO analysis was performed to select the top 10 terms in the categories of biological processes (BP), cell composition (CC), and molecular functions (MF). The BP category included terms such as 'immune response', 'inflammatory response', and 'defense response against viruses', while the MF category highlighted terms such as 'MHC class II protein complex binding', 'MHC class II receptor activation', 'CXCR chemokine receptor binding', and 'chemokine activation' (FIG. 4B).

Similarly, the GSEA analysis of C5 category, including up-regulated and down-regulated DEGs, yielded results similar to the pattern observed in the GO analysis (Table 3 below).

**[Table 3]**

| **No.** | **GENESET** | **NES / p-value** | **GENE NUMBER** | **CORE ENRICHMENT GENE LIST** |
|---|---|---|---|---|
| 1 | GOBP_REGULATION_OF_MACROPHAGE_MIGRATION | 2.676 / 0.001 | 25 | CCL5, SLAMF8 |
| 2 | GOBP_RESPIRATORY_BURST | 2.626 / 0.001 | 17 | SLAMF8 |
| 3 | GOBP_PHAGOSOME_MATURATION | 2.634 / 0.002 | 23 | SLAMF8 |
| 4 | GOBP_REGULATION_OF_NEUTROPHIL_MIGRATION | 2589 / 0.002 | 30 | SLAMF8, CD74 |
| 5 | GOBP_NEGATIVE_REGULATION_OF_CHEMOTAXIS | 2387 / 0.003 | 26 | CCL2, SLAMF8 |
| 6 | GOBP_MACROPHAGE_CHEMOTAXIS | 2.600 / 0.004 | 25 | CCL5, CCL2, SLAMF8,SAA1 |
| 7 | GOBP_TYPE_2_IMMUNE_RESPONSE | 2.593 / 0.006 | 17 | RSAD2, IDO1, CD74, IL6 |
| 8 | GOMF_MHC_CLASS_II_PROTEIN_COMPLEX_BINDING | 2.582 / 0.006 | 21 | HLA-DMB, HLA-DRB5, HLA-DQA2, HLA-DQB1, HLA-DPA1, HLA-DRA, CD74, HLA-DRB1, HLA-D 0A, HLA-DQA1, HLA-DMA, HLA-DPB1 |
| 9 | GOMF_MHC_PROTEIN_COMPLEX_BINDING | 2.644 / 0.007 | 24 | HLA-DMB, HLA-DRB5, HLA-DQA2, HLA-DQB1, HLA-DPA1, HLA-DRA, CD74, HLA-DRB1, HLA-DOA, HLA-DQA1, HLA-DMA, HLA-DPB1 |
| 10 | GOBP_NEGATIVE_REGULATION_OF_DNA_DAMAGE_RESPONSE_SIGNAL_TRANSDUCTION_BY_P53_CLASS_MEDIATOR | 2.621 / 0.007 | 15 | CD74 |

In particular, it was confirmed that the gene set listed in Table 3 above includes CCL5, CCL2, SLAMF8, RSAD2, IDO1, CD74, and HLA-DRB, which were identified as core enhancement genes among the top 10 upregulated DEGs. In addition, RNA-seq analysis identified 28 genes related to immunomodulation that showed higher expression in priming MSCs than in pure MSCs (Table 3). These genes, including CXCL10, CXCL9, CXCL11, CXCL2, CCL20, SERPINB7, IDO1, and PTGES, play important roles in inflammatory responses ranging from the recruitment and activation of immune cells to immunosuppression and tissue regeneration, and are essential for maintaining the balance between inflammation and regeneration processes. It was confirmed that most of these genes showed a pattern of increased expression in MSCs upon simultaneous treatment with IFN-γ and IL-β compared to treatment with IFN-γ alone.

Therefore, these results indicate that priming MSCs co-treated with IL-1β/IFN-γ interact with key factors identified in upregulated DEGs, exhibiting enhanced functionality that can significantly influence various disease states, including infection and inflammation.

### Example 3: Confirmation of lung tissue recovery and immune response modulation ability of priming MSCs co-treated with IL-1β/IFN-γ in vivo in ARDS

The LPS-induced lung injury model is widely used to study ARDS in rodents and can reproduce the neutrophilic inflammatory response observed in ARDS patients. Injecting LPS into the lungs of rodents can induce direct lung damage, which damages the alveolar epithelium.

Based on these principles, IL-1β/IFN-γ priming MSCs or pure MSCs were administered to an LPS-induced ARDS mouse model. To confirm the therapeutic effect thereof, lung tissue and bronchoalveolar lavage (BALF) samples were collected and the protein levels of IL-6 and TNF-α were analyzed.

First, as can be seen in FIGs. 5B and 5D, a visual inspection revealed severe congestion in the control group compared to the normal group. In contrast, when pure MSC or IL-1β/IFN-γ priming MSC was administered, pulmonary congestion was partially reduced, confirming that MSC can alleviate LPS-induced lung damage (FIGs. 5B and 5D).

The results of the histopathological analysis were also similar to those of the gross examination. As can be seen in FIGs. 5C and 5E, severe pathological changes such as congestion, necrosis, alveolar wall thickening, and immune cell infiltration were observed in the control group due to LPS, but these pathological changes were significantly alleviated in the group administered IL-1β/IFN-γ priming MSC or pure MSC, and in particular, IL-1β/IFN-γ priming MSC showed a potent therapeutic effect compared to pure MSC (FIGs. 5C and 5E).

Further analysis of BALF results showed that inflammatory cytokines IL-6 and TNF-α were increased in LPS-treated ARDS mice, as can be seen in FIGs. 5F and 5G. However, the levels of the cytokines were significantly reduced by MSC administration, and in particular, IL-1β/IFN-γ priming MSCs showed a significantly superior effect in reducing IL-6 and TNF-α in BALF (FIGs. 5F and 5G).

These results demonstrate that IL-1β/IFN-γ priming MSCs provide superior therapeutic effects compared to pure MSCs in an ARDS mouse model.

### Example 4: Spatial transcriptome profile analysis of IL-1β/IFN-γ priming MSCs in an LPS-induced ARDS mouse model

In an LPS-induced ARDS mouse model, gene expression patterns were analyzed through spatial transcriptome analysis to explain the mechanism and therapeutic effects of pure MSCs or IFN-γ/IL-1β priming MSCs on immune cells within damaged lung tissue. In the present analysis, human cells and mouse cells were distinguished through cluster analysis.

As can be seen in FIG. 6A, the analysis results confirmed that both pure MSCs and IFN-γ/IL-1β priming MSCs were present around the damaged lung tissue, but not in the control group (FIG. 6A). In particular, it was found that IFN-γ/IL-1β co-treated priming MSCs were present in higher quantities than pure MSCs.

In addition, it was confirmed that, in IL-1β/IFN-γ priming MSCs, gene expression related to metabolism, leukocyte migration, and blood flow was significantly increased while immune activity was decreased (FIG. 6B).

Consequently, it can be confirmed that IL-1β/IFN-γ priming MSCs have the potential to effectively treat the lungs in an LPS-induced ARDS model.

Next, the therapeutic mechanism was identified by investigating mouse gene expression patterns through spatial transcriptome analysis of lung tissue. Specifically, immune and stromal cell types were identified through cell type abundance analysis using xCell.

As a result, as can be seen in FIG. 6C, the expression of Treg-related genes was higher in the IFN-γ/IL-1β priming MSC group than in the control group (FIG. 6C). It was confirmed that gene expression related to macrophages, monocytes, and neutrophils was high in the control group, but the expression decreased after administration of pure MSCs or IFN-γ/IL-1β priming MSCs (FIG. 6C). This decrease was particularly evident in the regions where human genes were expressed. In addition, the reduction in immune cells and inflammation was greater in the IFN-γ/IL-1β priming MSC group than in the pure MSC group.

In the additional cell type analysis, as can be seen in FIG. 6D, it was confirmed that the expression of B cells, macrophages, monocytes, and neutrophils was high in the control group, but the expression of these cell types decreased after administration of pure MSCs or IFN-γ/IL-1β priming MSCs (FIG. 6D). In particular, the IL-1β/IFN-γ priming MSC group was more effective in inhibiting the activity of immune cells such as B cells and T cells. That is, through the above, it was confirmed that IL-1β/IFN-γ priming MSCs exhibit a therapeutic effect by effectively suppressing immune activity and inflammation-related cells in ARDS lung damage tissue.

### Example 5: Identification of key active factors for priming MSCs co-treated with IL-1β/IFN-γ

The major therapeutic effects and key mediators of IL-1β/IFN-γ priming MSCs in the treatment of ARDS were identified.

As a result, as can be seen in FIGs. 7A and 7B, it was confirmed through the Spatial Feature Plot (FIG. 7A) and VlnPlot (FIG. 7B) that the top four genes, Uba52, CXcl9, Cxcl5, and Cfb, whose expression was significantly increased in the control group, were inhibited by IL-1β/IFN-γ priming MSCs. These genes (Uba52, CXcl9, Cxcl5, and Cfb) are closely related to inflammatory processes, immune responses, and chemokine activity.

In addition, during the process of identifying the major mediators of IL-1β/IFN-γ priming MSCs, it was confirmed that the elastin (ELN) gene was significantly upregulated. Elastin is an essential component of the extracellular matrix and plays an important role in maintaining tissue elasticity and integrity. It is particularly important for lung function and contributes to forming the structural framework necessary for proper respiratory action. In particular, elastin is involved in the tissue regeneration and recovery process, and the significant increase in ELN expression in priming MSCs indicates that it may play an important role in regeneration and anti-inflammatory effects in cases of lung injury or dysfunction.

### Example 6: Spatial transcriptome dataset analysis to confirm immunomodulatory efficacy of pure MSCs and priming MSCs co-treated with IL-1β/IFN-γ

As a result of comparing differentially expressed genes (DEGs) in lung tissues of the IL-1β/IFN-γ priming MSC treatment group and the control group through volcano plot analysis, a total of 753 DEGs were identified, as can be seen in FIG. 8A (FIG. 8A). These DEGs showed an up-regulated or down-regulated expression pattern.

In addition, GO analysis of differentially expressed genes (DEGs) revealed significant gene groups in several pathways, as can be seen in FIG. 8B (FIG. 8B). The biological processes (BP) category included a particularly large number of pathways related to the regulation of innate immune responses, cytokine-mediated signaling, and type II interferon responses, suggesting that MSCs have the potential to regulate immune and inflammatory responses. In the cell composition (CC) category, an increase in genes related to the extracellular matrix, including collagen, was observed, suggesting the potential for activation of tissue repair mechanisms. Furthermore, molecular function (MF) analysis confirmed that genes related to growth factor binding and extracellular matrix structural components were up-regulated, while genes related to cytokine receptor binding and chemokine/cytokine activity were down-regulated, highlighting the immunosuppressive and tissue repair potential of IL-1β/IFN-γ primed MSCs.

These results suggest that MSCs primed with IL-1β/IFN-γ exhibit excellent capabilities in regulating immune responses and tissue repair mechanisms.

### Example 7: Spatial transcriptome dataset analysis to confirm therapeutic mechanism of pure MSCs and priming MSCs co-treated with IL-1β/IFN-γ

The therapeutic mechanism of pure MSCs and priming MSCs co-treated with IL-1β/IFN-γ was analyzed as follows. Specifically, the signaling pathway was analyzed using KEGG pathway reinforcement analysis (FIG. 9A). To confirm the therapeutic mechanism of pure MSCs and MSCs primed with IL-1β/IFN-γ, the NF-κB and TNF signaling pathways, which play important roles in ARDS pathology, were identified (FIG. 9B). As a result of NF-κB pathway analysis, the expression levels of Ccl4, Nfkbia, IL1b, Cd14, Cxcl12, Bcl2a1b, and Ccl21 in the pure MSC group were lower than those in the LPS control group (FIG. 10A). In the priming MSC group, inflammatory genes such as Ccl4, TNF, IL1β, Vcam1, Cxcl3, Cxcl1, Icam1, Cxcl12, and Ccl19 were more extensively down-regulated.

TNF pathway analysis results showed that the levels of Nfkbia, IL1b, Tnfrsf1b, Cxcl5, Cxcl10, Ccl5, Cebpb, and Ifi47 in the pure MSC group were lower than in the LPS control group (FIG. 10B). In the priming MSC group, a more pronounced reduction in inflammation-related genes, including Irf1, TNF, IL1b, Vcam1, Cxcl5, Cxcl3, Cxcl1, Cxcl10, Ccl5, Ccl2, Icam1, and Ifi47, was observed compared to the pure MSC group.

These results suggest that MSCs primed with IL-1β/IFN-γ offer enhanced therapeutic potential by regulating important inflammatory pathways in ARDS.

In addition, MSCs primed with IL-1β/IFN-γ showed inhibitory effects on MAC-1, LFA-1, and TNFR2 in the neutrophil extracellular trap (NET) formation signaling pathway (FIG. 10C). These results also demonstrate that MSCs primed with IL-1β/IFN-γ effectively reduce immune cell activation and neutrophil infiltration by suppressing inflammatory genes, demonstrating their potential to alleviate excessive inflammatory responses in ARDS.

In summary, it was confirmed that treatment with IL-1β, IFN-γ, or a combination thereof effectively enhances T cell proliferation inhibition and M2 macrophage activation, and by inhibiting TNF-α secretion and promoting IL-10 secretion within macrophages, effectively improves the immunomodulatory ability of umbilical cord blood-derived MSCs, thereby ultimately enabling their effective use in the prevention or treatment of ARDS. In particular, it was confirmed that the combined treatment effectively improves immunomodulatory ability compared to the stand-alone treatments, making it more effective in the treatment of ARDS.

In addition, it was confirmed that IL-1β, IFN-γ, or a combined treatment thereof increased the expression of CXCL1, CXCL2, CXCL3, CXCL5, CXCL6, and CXCL8, which play a role in neutrophil induction, and CXCL9, CXCL10, and CXCL11, which play a role in T cell induction, and increased the expression of CCL2 and CCL5, which promote monocyte and macrophage induction, and CCL20, which promotes lymphocyte induction. Further, the expression of all CXCL and CCL family genes except CCL5 increased when IFN-γ and IL-1β were co-treated compared to when IFN-γ was treated alone, and that the expression of SERPIN family genes (SERPINB2, SERPINB7, SERPING1, and SERPINA9) was also increased. Furthermore, it was confirmed that expression of elastin was increased.

Through the above, it was confirmed that in models transplanted with priming MSCs, pneumonia tissue lesions were significantly improved compared to models transplanted with pure MSCs, and TNF-α and IL-6 levels in bronchoalveolar lavage fluid (BALF) were significantly reduced, thereby confirming that priming MSCs treated with IFN-γ and IL-1β, particularly those treated with the combination thereof, effectively enhance motility, immunomodulatory capacity, and tissue regeneration, making them more effective for the treatment of ARDS.

From the above description, those skilled in the art to which the present invention pertains would understand that the present invention can be implemented in other specific forms without changing its technical concept or essential features. Therefore, the embodiments described above should be construed as being exemplified and not limiting the present disclosure. The scope of the present invention should be interpreted to include all modifications or modified forms derived from the meaning and scope of the claims as set forth below, as well as equivalent concepts, rather than from the detailed description above.

## Claims

1. A composition for enhancing immunomodulatory ability of a priming mesenchymal stem cell, comprising IL-1β, IFN-γ, or a combination thereof as an active ingredient.

2. The composition of claim 1, wherein the mesenchymal stem cell is an umbilical cord blood-derived mesenchymal stem cell.

3. The composition of claim 2, wherein the umbilical cord blood-derived mesenchymal stem cell is derived from a human.

4. The composition of claim 1, wherein the IL-1β and IFN-γ are comprised at concentrations of 2 ng/mL to 50 ng/mL and 10 ng/mL to 50 ng/mL, respectively.

5. The composition of claim 1, wherein the IL-1β and IFN-γ are treated for less than 48 hours.

6. The composition of claim 1, wherein the IL-1β, IFN-γ, or a combination thereof enhances immunomodulatory ability of the priming mesenchymal stem cell relative to that before treatment.

7. The composition of claim 6, wherein the immunomodulatory ability is immunosuppressive but not immunogenic.

8. The composition of claim 1, wherein the priming mesenchymal stem cell suppresses TNF-α secretion and promotes IL-10 secretion in macrophages.

9. The composition of claim 8, wherein the priming mesenchymal stem cell suppresses secretion of TNF-α to a range of 3,000 pg/mL to 4,500 pg/mL and promotes secretion of IL-10 to a range of 400 pg/mL to 700 pg/mL in macrophages.

10. The composition of claim 1, wherein the priming mesenchymal stem cell increases expression of an inflammation or immunoregulation-related gene.

11. The composition of claim 10, wherein the inflammation-related gene comprises one or more selected from CCL5, CCL2, SLAMF8, RSAD2, IDO1, CD74, HLA-DRB, and a combination thereof, and
the immunoregulation-related gene comprises one or more of CXCL10, CXCL9, CXCL11, CXCL2, CCL20, SERPINB7, IDO1, PTGES, and a combination thereof.

12. A pharmaceutical composition for preventing or treating acute respiratory distress syndrome, comprising the composition of any one of claims 1 to 11.

13. The pharmaceutical composition of claim 10, wherein the composition increases elastin to regenerate tissue or lung damage caused by acute respiratory distress syndrome.

14. The pharmaceutical composition of claim 12, wherein the composition increases serine protease inhibitor (SERPIN) superfamily gene expression to inhibit tissue and cellular damage resulting from neutrophil infiltration.

15. The pharmaceutical composition of claim 14, wherein the SERPIN superfamily gene is SERPINB2, SERPINB7, SERPING1, or SERPINA9.

16. The pharmaceutical composition of claim 12, wherein the composition further comprises a pharmaceutically acceptable carrier.

17. The pharmaceutical composition of claim 15, wherein the composition further comprises an excipient or an additive.

18. A method for preventing or treating acute respiratory distress syndrome, comprising administering the pharmaceutical composition of claim 12 to a subject other than a human.

19. A composition for preparing a priming mesenchymal stem cell having enhanced immunomodulatory ability, comprising IL-1β, IFN-γ, or a combination thereof as an active ingredient.

20. A method for preparing a priming mesenchymal stem cell having enhanced immunomodulatory ability, comprising treating a mesenchymal stem cell with IL-1β, IFN-γ,or a combination thereof.

21. A priming mesenchymal stem cell having enhanced immunomodulatory ability prepared by the method of claim 20.

22. A pharmaceutical composition for preventing or treating acute respiratory distress syndrome, comprising, as an active ingredient, a priming mesenchymal stem cell having enhanced immunomodulatory ability prepared by the method of claim 20.
